# EUROPEAN PATENT SPECIFICATION

(11) **EP 1 385 504 B1**
(45) Date of publication and mention of the grant of the patent: **17.12.2008**
(21) Application number: 02733968.8
(22) Date of filing: 10.04.2002
(51) Int. Cl.: A61K 31/35, A61P 17/02

(54) **METHOD OF INHIBITING ADHESION FORMATION**
VERFAHREN ZUR UNTERDRÜCKUNG VON ADHÄSIONSBILDUNG
METHODE D'INHIBITION DE FORMATION D'ADHERENCE

(30) Priority: 10.04.2001 US 282693 P
(43) Date of publication of application: 04.02.2004
(73) Proprietor: SMITHKLINE BEECHAM CORPORATION, Philadelphia, PA 19103 (US)
(72) Inventor: WILLETTE, Robert, N., King of Prussia, PA 19406 (US)
(74) Representative: Valentine, Jill Barbara
(86) International application number: PCT/US2002/011285
(87) International publication number: WO 2002/083125

(56) References cited:
- WO-A-98/14192
- WO-A1-98/14192

## Description

### Field of the Invention

This invention relates to the use of antagonists of the vitronectin receptor to inhibit adhesion formation.

### Background of the Invention

Integrins are a superfamily of cell adhesion receptors that couple intracellular cytoskeletal elements with extracellular matrix molecules. These cell surface adhesion receptors include αᵥβ₃ (the vitronectin receptor). The vitronectin receptor αᵥβ₃ is expressed on a number of cells, including endothelial, smooth muscle, osteoclast, and tumor cells, and, thus, it has a variety of functions. The αᵥβ₃ receptor expressed on the membrane of osteoclast cells mediates the adhesion of osteoclasts to the bone matrix, a key step in the bone resorption process. Ross, et al., J. Biol. Chem, 1987, 262, 7703. The αᵥβ₃ receptor expressed on human aortic smooth muscle cells mediates their migration into neointima, a process which can lead to restenosis after percutaneous coronary angioplasty. Brown, et al., Cardiovascular Res., 1994, 28, 1815. Additionally, Okada, et al., Am. J. Pathol., 1996, 149(1), 37 suggest that αᵥβ₃ plays a role in vascular integrity and remodeling following focal ischemia within an infarcted area.
WO98/14192 discloses a series of vitronectin receptor inhibitors which are stated to be useful in the treatment of inflammation, cancer, cardiovascular disorders and diseases wherein bone resorption is a factor, such as osteoporosis.

Surprisingly, it has been found that vitronectin receptor antagonists would be useful in inhibiting adhesion formation. In particular, the compounds of this invention are useful in the treatment of post-surgical adhesions.

### Summary of the Invention

The present invention provides a new method of treating post-surgical adhesion in a mammal, in particular a man, which comprises administering to a subject in need thereof an effective amount of a vitronectin receptor antagonist. of formula (I)

### Detailed Description of the Invention

The present invention relates to a therapeutic method for treating post-surgical adhesion The method utilizes the following class of antagonists which have been prepared and evaluated as effective vitronectin receptor antagonists:

Benzazepine ethers of the formula (I), which are described in PCT Application No. PCT/US97/18001, filed October 1, 1997, published as WO 98/14192 on April 9, 1998: 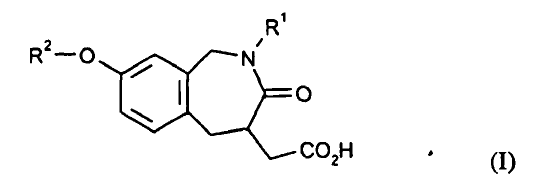 wherein:
R¹ is R⁷, or A-C₀₋₄alkyl, A-C₂₋₄alkenyl, A-C₂₋₄alkynyl, A-C₃₋₄oxoalkenyl, A-C₃₋₄oxoalkynyl, A-C₁₋₄aminoalkyl, A-C₃₋₄aminoalkenyl, A-C₃₋₄aminoalkynyl, optionally substituted by any accessible combination of one or more of R¹⁰ or R⁷;
A is H, C₃₋₆cycloalkyl, Het or Ar;
R⁷ is -COR⁸, -COCR'₂R⁹, -C(S)R⁸, -S(O)ₘOR', -S(O)ₘNR'R", -PO(OR'), -PO(OR')₂, -NO₂, or tetrazolyl;
each R⁸ independently is -OR', -NR'R", -NR'SO₂R', -NR'OR', or -OCR'₂CO(O)R';
R⁹ is -OR', -CN, -S(O)ᵣR', -S(O)ₘNR'₂, -C(O)R', C(O)NR'₂, or -CO₂R';
R¹⁰ is H, halo, -OR¹¹, -CN, -NR'R¹¹, -NO₂, -CF₃, CF₃S(O)ᵣ-, -CO₂R', -CONR'₂, A-C₀₋₆alkyl-, A-C₁₋₆oxoalkyl-, A-C₂₋₆alkenyl-, A-C₂₋₆alkynyl-, A-C₀₋₆alkyloxy-, A-C₀₋₆alkylamino- or A-C₀₋₆alkyl-S(O)ᵣ-;
R¹¹ is R', -C(O)R', -C(O)NR'₂, -C(O)OR', -S(O)ₘR', or -S(O)ₘNR'₂;
R² is 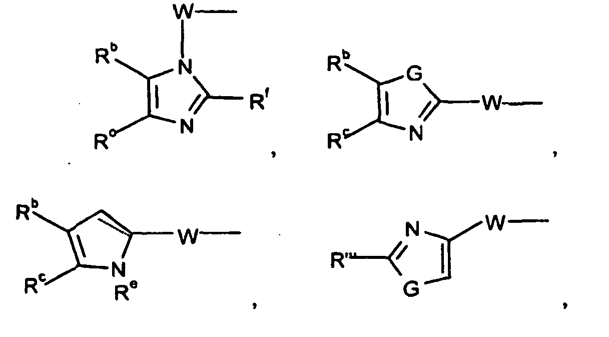 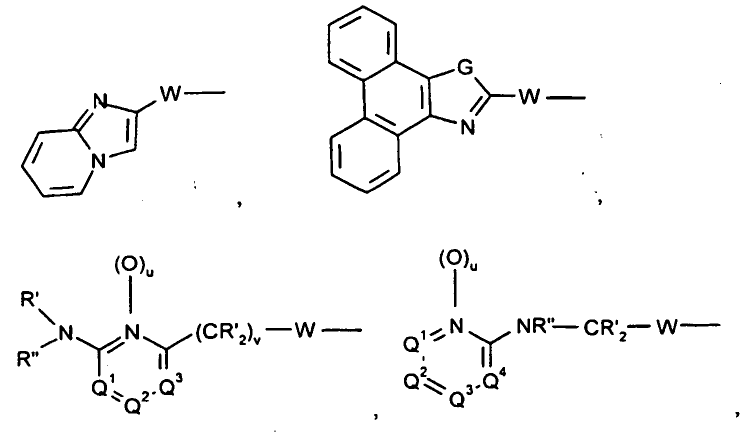 or 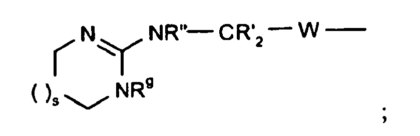
W is -(CHR^{g})ₐ-U- (CHR^{g})_{b}-;
U is absent or CO, CR^{g}₂, C(=CR^{g}₂), S(O)ₖ, O, NR^{g}, CR^{g}OR^{g}, CR^{g}(OR^{k})CR^{g}₂, CR^{g}₂CR^{g}(OR^{k}), C(O)CR^{g}₂, CR^{g}₂C(O), CONRⁱ, NRⁱCO, OC(O), C(O)O, C(S)O, OC(S), C(S)NR^{g}, NR^{g}C(S), S(O)₂NR^{g}, NR^{g}S(O)₂ N=N, NR^{g}NR^{g}, NR^{g}CR^{g}₂, CR^{g}₂NR^{g}, CR^{g}₂O, OCR^{g}₂, C≡C or CR^{g}=CR^{g};
G is NR^{e}, S or O;
R^{g} is H, C₁₋₆alkyl, Het-C₀₋₆alkyl, C₃-₇cycloalkyl-C₀₋₆alkyl or Ar-C₀₋₆alkyl;
R^{k} is R^{g}, -C(O)R^{g}, or -C(O)OR^{f};
Rⁱ is is H, C₁₋₆alkyl, Het-C₀₋₆alkyl, C₃₋₇cycloalkyl-C₀₋₆alkyl, Ar- C₀₋₆alkyl, or C₁₋₆alkyl substituted by one to three groups chosen from halogen, CN, NR^{g}₂, OR^{g}, SR^{g}, CO₂R^{g}, and CON(R^{g})₂;
R^{f} is H, C₁₋₆alkyl or Ar-C₀₋₆alkyl;
R^{e} is H, C₁₋₆alkyl, Ar-C₀₋₆alkyl, Het-C₀₋₆alkyl, C₃₋₇cycloalkyl-C₀₋₆alkyl, or (CH₂)ₖCO₂R^{g};
R^{b} and R^{c} are independently selected from H, C₁₋₆alkyl, Ar-C₀₋₆alkyl, Het-C₀₋₆alkyl, or C₃₋₆cycloalkyl-C₀₋₆alkyl, halogen, CF₃, OR^{f}, S(O)ₖR^{f}, COR^{f}, NO₂, N(R^{f})₂, CO(NR^{f})₂, CH₂N(R^{f})₂, or R^{b} and R^{c} are joined together to form a five or six membered aromatic or non-aromatic carbocyclic or heterocyclic ring, optionally substituted by up to three substituents chosen from halogen, CF₃, C₁₋₄alkyl, OR^{f}, S(O)ₖR^{f}, COR^{f}, CO₂R^{f}, OH_{,} NO_{2,} N(R^{f})₂, CO(NR^{f})₂, and CH₂N(R^{f})₂; or methylenedioxy;
Q¹, Q², Q³ and Q⁴ are independently N or C-R^{y}, provided that no more than one of Q¹, Q², Q³ and Q⁴ is N;
R' is H, C₁₋₆alkyl, Ar-C₀₋₆alkyl or C₃-₆cycloalkyl-C₀₋₆alkyl;
R" is R', -C(O)R' or -C(O)OR';
R"' is H, C₁₋₆alkyl, Ar-C₀₋₆alkyl, Het-C₀₋₆alkyl, or C₃₋₆cycloalkyl-C₀₋₆alkyl, halogen, CF₃, OR^{f}, S(O)ₖR^{f}, COR^{f}, NO₂, N(R^{f})₂, CO(NR^{f})₂, CH₂N(R^{f})₂;
R^{y} is H, halo, -OR^{g}, -SR^{g}, -CN, -NR^{g}R^{k}, -NO₂, -CF₃, CF₃S(O)ᵣ-, -CO₂R^{g}, -COR^{g} or -CONR^{g}₂, or C₁₋₆alkyl optionally substituted by halo, -OR^{g}, -SR^{g}, -CN, -NR^{g}R", -NO₂, -CF₃, R'S(O)ᵣ-, -CO₂R^{g}, -COR^{g} or -CONR^{g}₂;
a is 0, 1 or 2;
b is 0, 1 or 2;
k is 0, 1 or 2;
m is 1 or 2;
r is 0, 1 or 2;
s is 0, 1 or 2;
u is 0 or 1; and
v is 0 or 1;
or a pharmaceutically acceptable salt thereof.

Preferred formula (I) compounds used in the method of this invention are (S)-3-oxo-8-[3-(pyridin-2-ylamino)-1-propyloxy]-2-(2,2,2-trifluoroethyl)-2,3,4,5-tetrahydro-1H-2-benzazepine-4-acetic acid and (S)-8-[2-[6-(methylamino)pyridin-2-yl]-1-ethoxy]-3-oxo-2-(2,2,2-trifluoroethyl)-2,3,4,5-tetrahydro-1H-2-benzazepine-4-acetic acid, or pharmaceutically acceptable salts thereof.

In accordance with the present invention, it has been found that the administration of a vitronectin receptor antagonist to a surgical patient inhibits or ameliorates post-operative adhesion formation.

Surgical intervention involves wounding the patient in order to effect a cure. One unwanted result from surgery is post-operative adhesion formation. The term "adhesion" as used herein refers to conglutination, the process of adhering or uniting of two surfaces or parts. It has been reported that adhesion development is a major source of post-operative morbidity and mortality.

In the therapeutic use for the inhibition of adhesion formation, the vitronectin receptor antagonist is incorporated into standard pharmaceutical compositions. It can be administered orally, parenterally, rectally, topically or transdermally.

Pharmaceutical compositions of the vitronectin receptor antagonist may be formulated as solutions or lyophilized powders for parenteral administration. Powders may be reconstituted by addition of a suitable diluent or other pharmaceutically acceptable carrier prior to use. The liquid formulation may be a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

Alternately, the vitronectin receptor antagonist may be encapsulated, tableted or prepared in a emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier varies but, preferably, will be between about 20 mg to about 1 g per dosage unit. The pharmaceutical preparations are made following the conventional techniques of pharmacy involving milling, mixing, granulating, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

For rectal administration, the compounds of this invention may also be combined with excipients such as cocoa butter, glycerin, gelatin or polyethylene glycols and molded into a suppository.

The compound is administered either orally or parenterally to the patient, in a manner such that the concentration of drug is sufficient to be effective. The pharmaceutical composition containing the compound is administered at an oral dose of between about 0.1 to about 50 mg/kg in a manner consistent with the condition of the patient. Preferably the oral dose would be about 0.5 to about 20 mg/kg. For acute therapy, parenteral administration is preferred. An intravenous infusion of the peptide in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg. The compounds are administered one to four times daily at a level to achieve a total daily dose of about 0.4 to about 400 mg/kg/day. The precise level and method by which the compounds are administered is readily determined by one routinely skilled in the art by comparing the blood level of the agent to the concentration required to have a therapeutic effect.

No unacceptable toxicological effects are expected when eprosartan is administered in accordance with the present invention.

### Materials and Methods

The compounds of the instant invention are tested in known models of adhesion formation. These test systems include a rabbit sidewall model of adhesion formation as described in Rogers, et al., J. Invest. Surg., 9:388-391 (1996) and Rodgers, et al., Fertility and Surgery, 69(3):403-408 (1998); a rat model for adhesion formation as described in Harris, et al., Surgery, 117:663-669 (1995); and a rabbit animal model used to examine laparoscopic adhesion prevention. The rabbit uterine horn model may also be used to test the use of the instant vitronectin inhibiting compounds as inhibitors of adhesion formation. The experimental details and results using this model are detailed below.

### PROTOCOL:

Animals: New Zealand White rabbits, 2.4-2.7 kg, were purchased and quarantined for at least 2 days prior to use. The rabbits were randomized into appropriate control and treatment groups (see experimental designs below). The rabbits were housed on a 12:12 light:dark cycle with food and water available ad libitum. Each treatment group in all studies contained 8-10 animals.

Materials: The sutures that were used to close the peritoneum and skin were 4-0 Vicryl suture (Ethicon, Somerville, NJ). COMPOUND 1 is (S)-3-oxo-8-[3-(pyridin-2-ylamino)-1-propyloxy]-2-(2,2,2-trifluoroethyl)-2,3,4,5-tetrahydro-1H-2-benzazepine-4-acetic acid.

Adhesion Model: The animals received COMPOUND 1 or vehicle according to the experimental design ( below). The rabbits that received placebo at surgery received two doses of vehicle orally. Rabbits were anesthetized with a mixture of 55 mg/kg ketamine hydrochloride and 5 mg/kg Rompum intramuscularly. Following preparation for sterile surgery, a midline laparotomy was performed. The uterine horns were exteriorized and traumatized by abrasion of the serosal surface with gauze until punctate bleeding developed. Ischemia of both uterine horns was induced by removal of the collateral blood supply. The remaining blood supply to the uterine horns was the ascending branches of the utero-vaginal arterial supply of the myometrium. The midline muscle and skin incision were closed.

After 7 or 14 days, the rabbits were terminated and adhesions were scored on a site and rabbit basis. Specifically, the percentage of the area of the horns adherent to various organs was determined. In addition, the tenacity of the adhesions was scored using the following system:
- 0 =: No Adhesions
- 1 =: mild, easily dissectable adhesions
- 2 =: moderate adhesions; non-dissectable, does not tear the organ
- 3 =: dense adhesions; non-dissectable, tears organ when removed

An overall score which took into account all of the above data was given to each rabbit. The following scoring system was used:
0 No adhesions
0.5+ Light, filmy pelvic adhesions involving only one organ, typically only 1 or 2 small adhesions
1.0+ Light, filmy adhesions, not extensive although slightly more extensive than 0.5
1.5+ Adhesions slightly tougher and more extensive than a 1 rating
2.0+ Tougher adhesions, a little more extensive, uterine horns usually have adhesions to both bowel and bladder
2.5+ Same as 2, except the adhesions are usually not filmy at any site and more extensive
3.0+ Tougher adhesions than 2, more extensive, both horns are attached to the bowel and bladder, some movement of the uterus possible
3.5+ Same as 3, but adhesions slightly more extensive and tougher
4.0+ Severe adhesions, both horns attached to the bowel and bladder, unable to move the uterus without tearing the adhesions

The rabbits were scored by two independent observers that were blinded to the prior treatment of the animal. If there was disagreement as to the score to be assigned to an individual animal, the higher score was given.

Statistical Analysis: The tenacity and overall scores were analyzed by rank order analysis and analysis of variance on the ranks. The percentage area of the horns involved to the various organs were compared by Student's t test.

### Experimental designs:

Postoperatively received two loading doses of 60 mg/kg COMPOUND 1 orally prior to surgery. At the end of the procedure, the animals received either nothing (surgical control), or 12 ml of placebo (10% CMC) or one of two doses (1 mg/ml or 0.1 mg/ml) of COMPOUND 1 at the site of surgical injury.

*Local delivery study:* via osmotic minipump - initial validation study. Dose: 0.1 and 1.0 mM (10 ul/hr for 7 days). COMPOUND 1 was administered locally at the site of uterine injury by an Alzet miniosmotic pump . A polyethylene catheter (Clay Adams polyethylene tubing PE-60 ID 0.76 mm (0.030") OD 1.22 mm (0.048")) was introduced into the peritoneal cavity and sutured to the sidewall with 5-0 Ethilon immediately following uterine injury. The catheter was then attached to the pump and the midline muscle incision was be closed around the catheter. The pump was filled with 0.1 or 1 mM COMPOUND 1 (delivered at 10 µl/hour for 7 days) and placed in the subcutaneous space. The vehicle used to administer the drug was 8% cyclodextrin for the high dose and 0.8% for the low dose. Eight percent cyclodextrin was used in minipumps implanted into the control animals. Animal were sacrificed on day 7 for adhesion assessment.

*Oral administration study*: Prior to surgery, the rabbits received two loading doses of COMPOUND 1 (60 mg/kg, 5 mg/ml in 0.1 N NaOH) orally 24 and 48 hours prior to surgery. Immediately before surgery, the rabbits received one additional 60 mg/kg dose. The animals then received 60 mg/kg COMPOUND 1 daily until necropsy on day 14 after uterine surgery. Controls rabbits received vehicle by the same schedule.

*Oral + local study:* Two loading doses of COMPOUND 1 (60 mg/kg, po) were administered orally prior to surgery. Following surgery 12 ml of a viscous solution containing SB 267268 (1 or 0.1 mg/ml in 10% CMC) was introduced at the surgical site prior to closing the wound. The rabbits that received placebo at surgery received two doses of vehicle orally followed by 12 ml of placebo (10% CMC) at the surgical site. Surgical controls received no treatments before or after the surgical procedure. Animals were sacrificed at 7 day and 14 days

### RESULTS:

SAMPLE DATA SET TAKEN FROM THE ORAL + GEL COMPOUND 1 STUDY.

**Table 1. Data from Control Animals, 2 Week Necropsy Time**

| % Horn Involved | | | | | | | | |
|---|---|---|---|---|---|---|---|---|
| | Right Horn | | | | Left Horn | | | |
| Bowel | Bladder | Itself | Left | Bowel | Bladder | Itself | Right | Overall |
| 40(2) | 10(1) | 30(2) | 30(2) | 40(2) | 10(1) | 50(3) | 30(2) | 3.5 |
| - | 50(2) | 40(2) | 50(2) | - | 50(2) | 30(2) | 50(2) | 3.0 |
| - | 40(3) | 50(2) | 50(2) | - | 40(3) | 50(2) | 50(2) | 3.5 |
| 30(1) | 70(1) | 30(1) | 30(1) | 30(1) | 70(1) | 30(1) | 30(1) | 3.0 |
| 40(1) | - | 50(2) | 20(1) | 40(1) | - | 50(2) | 20(1) | 2.5 |
| 40(2) | - | 30(1) | 20(1) | 40(2) | - | 40(1) | 20(1) | 2.5 |
| 30(1) | 40(1) | 30(1) | 30(2) | 30(1) | 40(1) | 30(1) | 30(2) | 3.0 |
| 10(1) | 30(1) | 30(1) | 40(2) | 10(1) | 30(1) | 50(1) | 40(2) | 2.5 |
| 40(2) | 30(1) | 40(1) | 50(1) | 40(2) | 30(1) | 40(1) | 50(1) | 3.0 |
| 20(1) | 30(1) | 40(2) | 50(2) | 20(1) | 30(1) | 60(1) | 50(2) | 3.0 |
| 25.0±5.2 | 30.0±7.0 | 37.0±2.6 | 37.0±4.0 | 25.0±5.2 | 30.0±7.0 | 43.0±3.4 | 37.0±4.0 | 66.5±2.8 |

**Table 2. Data from Placebo Control Animals, 2 Week Necropsy Time**

| % Horn Involved | | | | | | | | |
|---|---|---|---|---|---|---|---|---|
| | Right Horn | | | | Left Horn | | | |
| Bowel | Bladder | Itself | Left | Bowel | Bladder | Itself | Right | Overall |
| 30(2) | 50(1) | 40(2) | 40(2) | 30(2) | 50(1) | 40(3) | 40(2) | 3.5 |
| 30(1) | 10(1) | 40(2) | 30(1) | 30(1) | 10(1) | 20(1) | 30(1) | 2.0 |
| - | 30(1) | 10(1) | 30(1) | - | 30(1) | - | 30(1) | 1.5 |
| - | 20(1) | - | 10(1) | - | 20(1) | 20(1) | 10(1) | 1.5 |
| 40(1) | 40(1) | - | 40(2) | 40(1) | 40(1) | 40(2) | 40(2) | 3.0 |
| - | 50(1) | 10(1) | 30(1) | - | 50(1) | 30(1) | 30(1) | 2.0 |
| 30(1) | 20(1) | 30(1) | 40(2) | 30(1) | 20(1) | 10(1) | 40(2) | 2.5 |
| 40(1) | 10(1) | - | 30(1) | 40(1) | 10(1) | 10(1) | 30(1) | 2.0 |
| - | - | 30(1) | 30(1) | - | - | 40(1) | 30(1) | 1.5 |
| - | 40(1) | 40(1) | 40(1) | - | 40(1) | - | 40(1) | 2.0 |
| 17.0±5.8 | 27.0±5.6 | 20.0±5.6 | 32.0±2.9 | 17.0±5.8 | 27.0±5.6 | 21.0±5.0 | 32.0±2.9 | 45.2±5.9 |

**Table 3. Data from Treated Animals, 1 mg/ml COMPOUND 1, 2 Week Necropsy Time**

| % Horn Involved | | | | | | | | |
|---|---|---|---|---|---|---|---|---|
| | Right Horn | | | | Left Horn | | | |
| Bowel | Bladder | Itself | Left | Bowel | Bladder | Itself | Right | Overall |
| 10(1) | 10(1) | - | - | - | - | - | - | 0.5 |
| 30(1) | - | 40(1) | - | 30(1) | - | 40(1) | - | 2.0 |
| - | - | 30(1) | - | - | - | 30(1) | - | 1.0 |
| 10(1) | - | 20(1) | - | - | - | 20(1) | - | 1.0 |
| 30(2) | - | 30(2) | 30(2) | 30(2) | - | 30(2) | 30(2) | 2.5 |
| - | - | - | - | 30(1) | - | 10(1) | - | 1.0 |
| 10(1) | 10(1) | 10(1) | - | 10(1) | - | - | - | 1.0 |
| - | - | - | - | 40(2) | 20(2) | 10(2) | - | 1.5 |
| 10(1) | 20(1) | - | - | - | 20(1) | 20(1) | - | 1.0 |
| 30(1) | - | - | 20(1) | 30(1) | - | 20(1) | 20(1) | 1.5 |
| 13.0±4.0 | 4.0±2.2 | 13.0±5.0 | 5.0±3.4 | 17.0±5.2 | 4.0±2.7 | 18.0±4.2 | 5.0±3.4 | 21.2±5.5 |

**Table 4. Data from Treated Animals, 0.1 mg/ml COMPOUND 1, 2 Week Necropsy Time**

| % Horn Involved | | | | | | | | |
|---|---|---|---|---|---|---|---|---|
| | Right Horn | | | | Left Horn | | | |
| Bowel | Bladder | Itself | Left | Bowel | Bladder | Itself | Right | Overall |
| 20(1) | 50(1) | 30(2) | 10(1) | 20(1) | 50(1) | 30(2) | 10(1) | 2.5 |
| - | - | - | 40(1) | - | - | 20(1) | 40(1) | 1.5 |
| 10(1) | - | 30(1) | 30(2) | 10(1) | - | 30(1) | 30(2) | 2.5 |
| - | - | 40(1) | 30(1) | - | - | 40(1) | 30(1) | 1.5 |
| - | 10(1) | 30(2) | 30(2) | 10(1) | 10(1) | 30(2) | 30(2) | 2.0 |
| 10(1) | 10(1) | 10(1) | 40(1) | - | 10(1) | - | 40(1) | 2.0 |
| 20(1) | - | - | - | 20(1) | - | 20(1) | - | 1.0 |
| - | 10(1) | - | - | - | - | 10(1) | - | 0.5 |
| 40(1) | - | - | 20(1) | 40(1) | - | 20(1) | 20(1) | 1.5 |
| - | 10(1) | 20(1) | 10(1) | - | - | 20(1) | 10(1) | 1.0 |
| 10.0±4.2 | 9.0±4.8 | 16.0±5.0 | 21.0±4.8 | 10.0±4.2 | 7.0±5.0 | 22.0 ±3.6 | 21.0±4.8 | 30.2±6.2 |

**Table 5. Data from Control Animals, 1 Week Necropsy Time**

| % Horn Involved | | | | | | | | |
|---|---|---|---|---|---|---|---|---|
| | Right Horn | | | | Left Horn | | | |
| Bowel | Bladder | Itself | Left | Bowel | Bladder | Itself | Right | Overall |
| 50(1) | 30(1) | 30(1) | 40(1) | 50(1) | 30(1) | 30(1) | 40(1) | 3.0 |
| 50(1) | 30(2) | 60(1) | 50(1) | 50(1) | 30(2) | 60(1) | 50(1) | 3.5 |
| 40(1) | 20(1) | 40(1) | 50(1) | 40(1) | 20(1) | 40(1) | 50(1) | 3.0 |
| 50(2) | 10(1) | 40(1) | 30(2) | 50(2) | 10(1) | 30(2) | 30(2) | 3.5 |
| 20(1) | 30(1) | 30(1) | 40(1) | 20(1) | 30(1) | 40(2) | 40(1) | 3.0 |
| 30(1) | 40(2) | 40(1) | 40(1) | 30(1) | 40(2) | 40(1) | 40(1) | 3.0 |
| 30(1) | 40(1) | 40(1) | 40(1) | 30(1) | 40(1) | 30(1) | 40(1) | 2.5 |
| 50(2) | - | 30(1) | 40(2) | 50(2) | - | 50(1) | 40(2) | 3.0 |
| - | 40(1) | 50(1) | 40(2) | - | 40(1) | 50(1) | 40(2) | 3.0 |
| 30(2) | 30(1) | 50(2) | 40(2) | 30(2) | 30(1) | 50(2) | 40(2) | 3.0 |
| 35.0±5.2 | 27.0±4.2 | 41.0±3.1 | 41.0±1.8 | 35.0±5.2 | 27.0±4.2 | 42.0±3.3 | 41.0±1.8 | 69.1±2.1 |

**Table 6. Data from Placebo Control Animals, 1 Week Necropsy Time**

| % Horn Involved | | | | | | | | |
|---|---|---|---|---|---|---|---|---|
| | Right Horn | | | | Left Horn | | | |
| Bowel | Bladder | Itself | Left | Bowel | Bladder | Itself | Right | Overall |
| - | 30(1) | 40(1) | 20(1) | - | 30(1) | 40(1) | 20(1) | 2.0 |
| 20(1) | 30(1) | 40(1) | 30(1) | 20(1) | 30(1) | 40(1) | 30(1) | 2.5 |
| 20(1) | 30(1) | 30(1) | 40(1) | 20(1) | 30(1) | 20(1) | 40(1) | 2.0 |
| 60(1) | - | 30(1) | 30(1) | 60(1) | - | 30(1) | 30(1) | 3.0 |
| - | 40(2) | 30(1) | 10(1) | - | 40(2) | 50(1) | 10(1) | 2.5 |
| 20(1) | 10(1) | 10(1) | 20(1) | 20(1) | 10(1) | 30(1) | 20(1) | 2.0 |
| - | - | 30(1) | 30(1) | - | - | 30(1) | 30(1) | 1.5 |
| - | 20(1) | 40(2) | 50(1) | - | 20(1) | 40(2) | 50(1) | 2.5 |
| 40(1) | 20(1) | 40(2) | 30(2) | 40(1) | 20(1) | - | 30(2) | 2.5 |
| 30(1) | 10(1) | 30(1) | 20(1) | 30(1) | 10(1) | 30(1) | 20(1) | 2.0 |
| 19.0±6.4 | 19.0±4.3 | 32.0±2.9 | 28.0±3.6 | 19.0±6.4. | 19.0±4.3 | 31.0±4.3 | 28.0±3.6 | 48.7±3.9 |

**Table 7. Data from Treated Animals, 1 mg/ml COMPOUND 1, 1 Week Necropsy Time**

| % Horn Involved | | | | | | | | |
|---|---|---|---|---|---|---|---|---|
| | Right Horn | | | | Left Horn | | | |
| Bowel | Bladder | Itself | Left | Bowel | Bladder | Itself | Right | Overall |
| 30(1) | - | 40(1) | - | 30(1) | - | 40(1) | - | 2.0 |
| - | 10(1) | 10(1) | 30(1) | 20(1) | - | - | 30(1) | 1.5 |
| 30(1) | - | - | 30(1) | 30(1) | - | 30(1) | 30(1) | 1.5 |
| 30(1) | - | - | - | 30(1) | - | 20(1) | - | 1.0 |
| - | - | 20(1) | 20(1) | - | - | 20(1) | 20(1) | 1.0 |
| 10(1) | - | - | - | - | - | 30(1) | - | 0.5 |
| 20(1) | 10(1) | - | 10(1) | - | - | 30(1) | 10(1) | 1.5 |
| 30(1) | - | 20(1) | 30(1) | 30(1) | - | - | 30(1) | 1.5 |
| 20(1) | 10(1) | - | 20(2) | 20(1) | - | 10(1) | 20(2) | 1.5 |
| 20(1) | - | 10(1) | 10(1) | 20(1) | - | - | 10(1) | 1.0 |
| 19.0±3.8 | 3.0±1.5 | 10.0±4.2 | 15.0±4.0 | 18.0±4.2 | 0.0±0.0 | 18.0±4.7 | 15.0±4.0 | 21.3±3.9 |

**Table 8. Data from Treated Animals, 0.1 mg/ml COMPOUND 1, 2 Week Necropsy Time**

| % Horn Involved | | | | | | | | |
|---|---|---|---|---|---|---|---|---|
| | Right Horn | | | | Left Horn | | | |
| Bowel | Bladder | Itself | Left | Bowel | Bladder | Itself | Right | Overall |
| - | - | 10(1) | - | 30(1) | - | 40(1) | - | 1.5 |
| - | 20(1) | - | 20(1) | - | 20(1) | 30(1) | 20(1) | 1.5 |
| 10(1) | - | 10(1) | 30(1) | - | - | - | 30(1) | 1.0 |
| 40(1) | 30(1) | 30(1) | - | 40(1) | 30(1) | 30(1) | - | 2.0 |
| 30(1) | 10(1) | - | 10(1) | 30(1) | 10(1) | 30(1) | 10(1) | 1.5 |
| 10(1) | 10(1) | 10(1) | 40(1) | 10(1) | 10(1) | - | 40(1) | 1.5 |
| 30(1) | - | 10(1) | 30(1) | 30(1) | - | 50(1) | 30(1) | 2.0 |
| - | - | 30(1) | - | - | - | 20(1) | - | 1.0 |
| - | - | 10(1) | 20(1) | - | - | - | 20(1) | 0.5 |
| 10(1) | - | - | - | 10(1) | - | 30(1) | - | 0.5 |
| 13.0±4.7 | 7.0±3.4 | 11.0±3.5 | 15.0±4.8 | 15.0±5.0 | 7.0±3.4 | 23.0±5.6 | 15.0±4.8 | 22.0±4.8 |

**Table 9. Summary of Adhesion Incidence Data**

| Group | # Sites Adhesion Free | % Sites Adhesion Free |
|---|---|---|
| Control 2 Weeks | 8 | 10.0 |
| Placebo 2 Weeks | 17 | 21.25 |
| 1 mg/ml COMPOUND 1 | 45 | 56.25 |
| 0.1 mg/ml COMPOUND 1 | 31 | 38.75 |
| | | |
| Control 1 Week | 4 | 5.0 |
| Placebo 1 Week | 13 | 16.25 |
| 1 mg/ml COMPOUND 1 | 36 | 45.0 |
| 0.1 mg/ml COMPOUND 1 | 34 | 42.5 |

It is to be understood that the invention is not limited to the embodiment illustrated hereinabove and the right is reserved to the illustrated embodiment and all modifications coming within the scope of the following claims.

## Claims

1. The use of a compound of the formula (I): 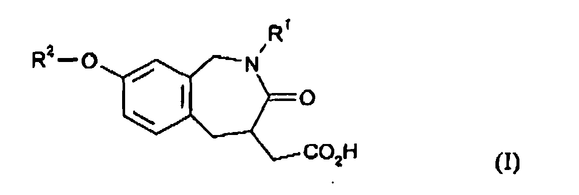 wherein:
R¹ is R⁷, or A-C₀₋₄alkyl, A-C₂₋₄alkenyl, A-C₂₋₄alkynyl, A-C₃₋₄oxoalkenyl, A-C₃₋₄oxoalkynyl, A-C₁₋₄aminoalkyl, A-C₃₋₄aminoalkenyl, A-C₃₋₄aminoalkynyl, optionally substituted by any accessible combination of one or more of R¹⁰ or R⁷;
A is H, C₃₋₆cycloalkyl, Het or Ar;
R⁷ is -COR⁸, -COCR'₂R⁹, -C(S)R⁸, -S(O)ₘOR', -S(O)ₘNR'R", -PO(OR'), -PO(OR')₂, -NO₂, or tetrazolyl;
each R⁸ independently is -OR', -NR'R", -NR'SO₂R', -NR'OR', or -OCR'₂CO(O)R';
R⁹ is -OR', -CN, -S(O)ᵣR', -S(O)ₘNR'₂, -C(O)R', C(O)NR'₂, or -CO₂R';
R¹⁰ is H, halo, -OR¹¹, -CN, -NR'R¹¹, -NO₂, -CF₃, CF₃S(O)ᵣ-, -CO₂R', -CONR'₂, A-C₀₋₆alkyl-, A-C₁₋₆oxoalkyl-, A-C₂₋₆alkenyl-, A-C₂₋₆alkynyl-, A-C₀₋₆alkyloxy-, A-C₀₋₆alkylamino- or A-C₀₋₆alkyl-S(O)ᵣ-;
R¹¹ is R', -C(O)R', -C(O)NR'₂, -C(O)OR', -S(O)ₘR', or -S(O)ₘNR'₂;
R² is 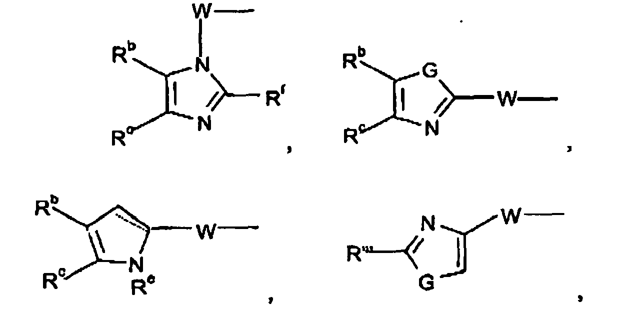 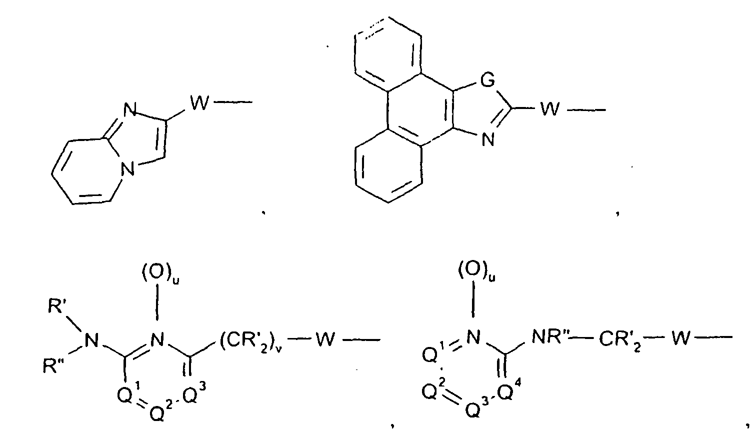 or 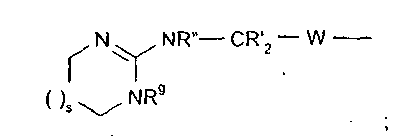
W is -(CHR^{g})ₐ-U- (CHR^{g})_{b}-;
U is absent or CO, CR^{g}₂, C(=CR^{g}₂), S(O)ₖ, O, NR^{g}, CR^{g}OR^{g}, CR^{g}(OR^{k})CR^{g}₂, CR^{g}₂CR^{g}(OR^{k}), C(O)CR^{g}₂, CR^{g}₂C(O), CONRⁱ, NRⁱCO, OC(O), C(O)O, C(S)O, OC(S), C(S)NR^{g}, NR^{g}C(S), S(O)₂NR^{g}, NR^{g}S(O)₂ N=N, NR^{g}NR^{g}, NR^{g}CR^{g}₂, CR^{g}₂NR^{g}, CR^{g}₂O, OCR^{g}₂, C≡C or CR^{g}=CR^{g};
G is NR^{e}, S or O;
R^{g} is H, C₁₋₆alkyl, Het-C₀₋₆alkyl, C₃₋₇cycloalkyl-C₀₋₆alkyl or Ar-C₀₋₆alkyl;
R^{k} is R^{g}, -C(O)R^{g}, or -C(O)OR^{f};
Rⁱ is H, C₁₋₆alkyl, Het-C₀₋₆alkyl, C₃₋₇cycloalkyl-C₀₋₆alkyl, Ar- C₀₋₆alkyl, or C₁₋₆alkyl substituted by one to three groups chosen from halogen, CN, NR^{g}₂, OR^{g}, SR^{g}, CO₂R^{g}, and CON(R^{g})₂;
R^{f} is H, C₁₋₆alkyl or Ar-C₀₋₆alkyl;
R^{e} is H, C₁₋₆alkyl, Ar-C₀₋₆alkyl, Het-C₀₋₆alkyl, C₃₋₇cycloalkyl-C₀₋₆alkyl, or (CH₂)ₖCO₂R^{g};
R^{b} and R^{c} are independently selected from H, C₁₋₆alkyl, Ar-C₀₋₆alkyl, Het-C₀₋₆alkyl, or C₃₋₆cycloalkyl-C₀₋₆alkyl, halogen, CF₃, OR^{f}, S(O)ₖR^{f}, COR^{f}, NO₂, N(R^{f})₂, CO(NR^{f})₂, CH₂N(R^{f})₂, or R^{b} and R^{c} are joined together to form a five or six membered aromatic or non-aromatic carbocyclic or heterocyclic ring, optionally substituted by up to three substituents chosen from halogen, CF₃, C₁₋₄alkyl, OR^{f}, S(O)ₖR^{f}, COR^{f}, CO₂R^{f}, OH, NO₂, N(R^{f})₂, CO(NR^{f})₂, and CH₂N(R^{f})₂; or methylenedioxy;
Q¹, Q², Q³ and Q⁴ are independently N or C-RY, provided that no more than one of Q¹, Q², Q³ and Q⁴ is N;
R' is H, C₁₋₆alkyl, Ar-C₀₋₆alkyl or C₃₋₆cycloalkyl-C₀₋₆alkyl;
R" is R', -C(O)R' or -C(O)OR';
R"' is H, C₁₋₆alkyl, Ar-C₀₋₆alkyl, Het-C₀₋₆alkyl, or C₃₋₆cycloalkyl-C₀₋₆alkyl, halogen, CF₃, OR^{f}, S(O)ₖR^{f}, COR^{f}, NO₂, N(R^{f})₂, CO(NR^{f})₂, CH₂N(R^{f})₂;
R^{y} is H, halo, -OR^{g}, -SR^{g}, -CN, -NR^{g}R^{k}, -NO₂, -CF₃, CF₃S(O)ᵣ-, -CO₂R^{g}, -COR^{g} or -CONR^{g}₂, or C₁₋₆alkyl optionally substituted by halo, -OR^{g}, -SR^{g}, -CN, -NR^{g}R", -NO₂, -CF₃, R'S(O)ᵣ-, -CO₂R^{g}, -COR^{g} or -CONR^{g}₂;
a is 0, 1 or 2;
b is 0, 1 or 2;
k is 0, 1 or 2;
m is I or 2;
r is 0, 1 or 2;
s is 0, 1 or 2;
u is 0 or 1; and
v is 0 or 1;
or a pharmaceutically acceptable salt thereof,
in the manufacture of a medicament for treating post-surgical adhesions.

2. The use according to claim 1 wherein the compound is (S)-3-oxo-8-[3-(pyridin-2-ylamino)-1-propyloxy]-2-(2,2,2-trifluoroethyl)-2,3,4,5-tetrahydro-1H-2-benzazepine-4-acetic acid or a pharmaceutically acceptable salt thereof.

3. The use according to claim 1 wherein the compound is (S)-8-[2-[6-(methylamino)pyridin-2-yl]-1-ethoxy]-3-oxo-2-(2,2,2-trifluoroethyl)-2,3,4,5-tetrahydro-1H-2-benzazepine-4-acetic acid or a pharmaceutically acceptable salt thereof.

4. The use of (S)-10,11-dihydro-3-[2-(5,6,7,8-tetrahydro-1,8-naphthyridin-2-yl)-1-ethoxy]-5H-dibenzo[a,d]cycloheptene-10-acetic acid, or a pharmaceutically acceptable salt thereof, in the manufacture of a medicament for the inhibition of adhesion formation.

## Patentansprüche

1. Verwendung einer Verbindung der Formel (I): 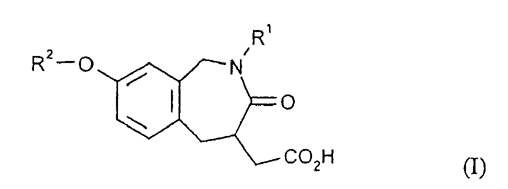 wobei:
R¹ für R⁷ oder A-C₀₋₄-Alkyl, A-C₂₋₄-Alkenyl, A-C₂₋₄-Alkinyl, A-C₃₋₄-Oxoalkenyl, A-C₃₋₄-Oxoalkinyl, A-C₁₋₄-Aminoalkyl, A-C₃₋₄-Aminoalkenyl, A-C₃₋₄-Aminoalkinyl, gegebenenfalls substituiert mit jeder erreichbaren Kombination einer oder mehrerer Reste R¹⁰ oder R⁷steht;
A für H, C₃₋₆-Cycloalkyl, Het oder Ar steht;
R⁷ für -COR⁸, -COCR'₂R⁹, -C(S)R⁸, -S(O)ₘOR', -S(O)ₘNR'R", -PO(OR'), -PO(OR')₂, -NO₂ oder Tetrazolyl steht;
jeder Rest R⁸ unabhängig für -OR', -NR'R", -NR'SO₂R', -NR'OR' oder -OCR'₂CO(O)R' steht;
R⁹ für -OR', -CN, -S(O)ᵣR', -S(O)ₘNR'₂, -C(O)R', C(O)NR'₂ oder -CO₂R' steht;
R¹⁰ für H, Halogen, -OR¹¹, -CN, -NR'R¹¹, -NO₂, -CF₃, CF₃S(O)ᵣ-, -CO₂R', -CONR'₂,
A-C₀₋₆-Alkyl-, A-C₁₋₆-Oxoalkyl-, A-C₂₋₆-Alkenyl-, A-C₂₋₆-Alkinyl-, A-C₀₋₆-Alkyloxy-, A-C₀₋₆-Alkylamino- oder A-C₀₋₆-Alkyl-S(O)ᵣ- steht;
R¹¹ für R', -C(O)R', -C(O)NR'₂, -C(O)OR', -S(O)ₘR' oder -S(O)ₘNR'₂ steht;
R² für 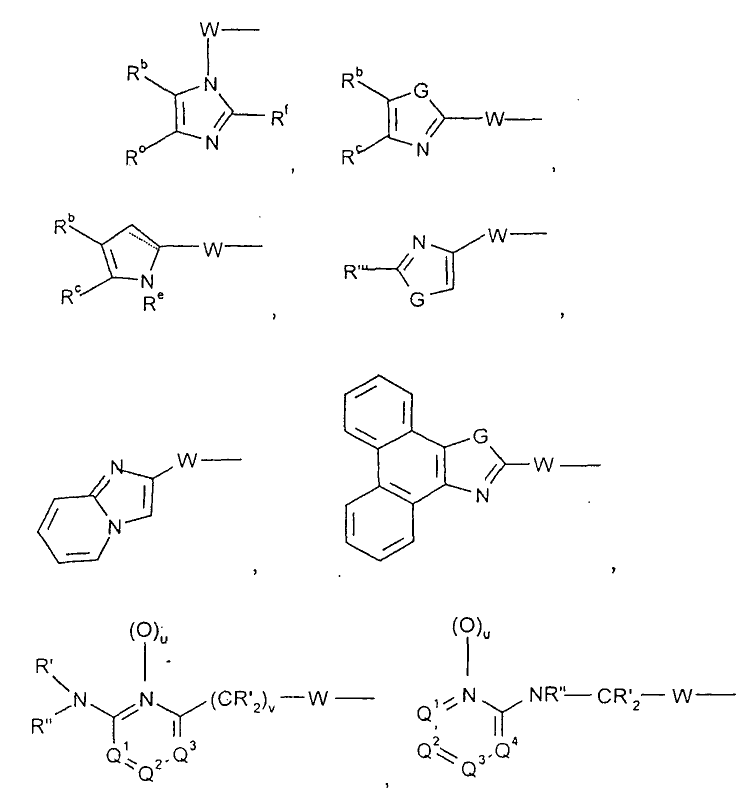 oder 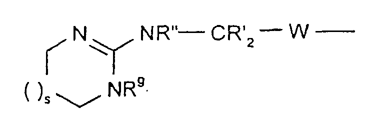 steht;
W für -(CHR^{g})ₐ-U-(CHR^{g})_{b}- steht;
U abwesend ist oder CO, CR^{g}₂, C(=CR^{g}₂), S(O)ₖ, O, NR^{g}, CR^{g}OR^{g}, CR^{g}(OR^{k})CR^{g}₂, _{.} CR^{g}₂CR^{g}(OR^{k}), C(O)CR^{g}₂, CR^{g}₂C(O), CONRⁱ, NRⁱCO, OC(O), C(O)O, C(S)O, OC(S), C(S)NR^{g}, NR^{g}C(S), S(O)₂NR^{g}, NR^{g}S(O)₂N=N, NR^{g}NR^{g}, NR^{g}CR^{g}₂, CR^{g}₂NR^{g}, CR^{g}₂O, OCR^{g}₂, C≡C oder CR^{g}=CR^{g} ist;
G für NR^{e}, S oder O steht;
R^{g} für H, C₁₋₆-Alkyl, Het-C₀₋₆-Alkyl, C₃₋₇-Cycloalkyl-C₀₋₆-alkyl oder Ar-C₀₋₆-Alkyl steht; R^{k} für R^{g}, -C(O)R^{g} oder -C(O)OR^{f} steht;
Rⁱ für H, C₁₋₆-Alkyl, Het-C₀₋₆-Alkyl, C₃₋₇-Cycloalkyl-C₀₋₆-alkyl, Ar-C₀₋₆-Alkyl oder C₁₋₆-Alkyl substituiert mit einem bis drei Resten, ausgewählt aus Halogen, CN, NR^{g}₂, OR^{g}, SR^{g}, CO₂R^{g} und CON(R^{g})₂, steht;
R^{f} für H, C₁₋₆-Alkyl oder Ar-C₀₋₆-Alkyl steht;
R^{e} für H, C₁₋₆-Alkyl, Ar-C₀₋₆-Alkyl, Het-C₀₋₆-Alkyl, C₃₋₇-Cycloalkyl-C₀₋₆-alkyl oder (CH₂)ₖCO₂R^{g} steht;
R^{b} und R^{c} unabhängig ausgewählt sind aus H, C₁₋₆-Alkyl, Ar-C₀₋₆-Alkyl, Het-C₀₋₆-Alkyl oder C₃₋₆-Cycloalkyl-C₀₋₆-alkyl, Halogen, CF₃, OR^{f}, S(O)ₖR^{f}, COR^{f}, NO₂, N(R^{f})₂, CO(NR^{f})₂, CH₂N(R^{f})₂, oder R^{b} und R^{c} miteinander verknüpft sind, um einen 5- oder 6-gliedrigen, aromatischen oder nicht-aromatischen, carbocyclischen oder heterocyclischen Ring zu bilden, gegebenenfalls substituiert mit bis zu 3 Substituenten, ausgewählt aus Halogen, CF₃, C₁₋₄-Alkyl, OR^{f}, S(O)ₖR^{f}, COR^{f}, CO₂R^{f}, OH, NO₂, N(R^{f})₂, CO(NR^{f})₂ und CH₂N(R^{f})₂; oder Methylendioxy;
Q¹, Q², Q³ und Q⁴ unabhängig N oder C-R^{y} darstellen mit der Maßgabe, dass nicht mehr als einer von Q¹, Q², Q³ und Q⁴ gleich N ist;
R' für H, C₁₋₆-Alkyl, Ar-C₀₋₆-Alkyl oder C₃₋₆-Cycloalkyl-C₀₋₆-alkyl steht;
R" für R', -C(O)R' oder-C(O)OR' steht;
R''' für H, C₁₋₆-Alkyl, Ar-C₀₋₆-Alkyl, Het-C₀₋₆-Alkyl oder C₃₋₆-Cycloalkyl-C₀₋₆-alkyl, Halogen, CF₃, OR^{f}, S(O)ₖR^{f}, COR^{f}, NO₂, N(R^{f})₂, CO(NR^{f})₂, CH₂N(R^{f})₂ steht;
R^{y} für H, Halogen, -OR^{g}, -SR^{g}, -CN, -N-R^{g}R^{k}, -NO₂, -CF₃, CF₃S(O)ᵣ-, -CO₂R^{g}, -COR^{g} oder -CONR^{g}₂, oder C₁₋₆-Alkyl gegebenenfalls substituiert mit Halogen, -OR^{g}, -SR^{g}, -CN, -NR^{g}R", -NO₂, -CF₃, R'S(O)ᵣ-, -CO₂R^{g}, -COR^{g} oder-CONR^{g}₂ steht;
a gleich 0, 1 oder 2 ist;
b gleich 0, 1 oder 2 ist;
k gleich 0, 1 oder 2 ist;
m gleich 1 oder 2 ist;
r gleich 0, 1 oder 2 ist;
s gleich 0, 1 oder 2 ist;
u gleich 0 oder 1 ist; und
v gleich 0 oder 1 ist;
oder ein pharmazeutisch verträgliches Salz davon
zur Herstellung eines Medikaments zur Behandlung von post-operativen Adhäsionen.

2. Verwendung nach Anspruch 1, wobei die Verbindung (S)-3-Oxo-8-[3-(pyridin-2-ylamino)-1-propyloxy]-2-(2,2,2-trifluorethyl)-2,3,4,5-tetrahydro-1H-2-benzazepin-4-essigsäure oder ein pharmazeutisch verträgliches Salz davon ist.

3. Verwendung nach Anspruch 1, wobei die Verbindung (S)-8-[2-[6-(Methylamino)pyridin-2-yl]-1-ethoxy]-3-oxo-2-(2,2,2-trifluorethyl)-2,3,4,5-tetrahydro-1H-2-benzazepin-4-essigsäure oder ein pharmazeutisch verträgliches Salz davon ist.

4. Verwendung von (S)-10,11-Dihydro-3-[2-(5,6,7,8-tetrahydro-1,8-naphthyridin-2-yl)-1-ethoxy]-5H-dibenzo[a,d]cyclohepten-10-essigsäure oder eines pharmazeutisch verträglichen Salzes davon zur Herstellung eines Medikaments zur Behandlung von post-operativen Adhäsionen.

## Revendications

1. Utilisation d'un composé de formule (I) : 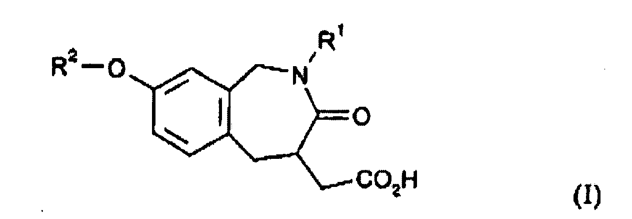 dans laquelle :
R¹ représente un groupe R⁷, ou A-(alkyle en C₀ à C₄), A-(alcényle en C₂ à C₄), A-(alcynyle en C₂ à C₄), A-(oxo-alcényle en C₃ ou C₄), A-(oxoalcynyle en C₃ ou C₄), A-(amino-alkyle en C₁ à C₄), A-(aminoalcényle en C₃ ou C₄), A-(aminoalcynyle en C₃ ou C₄), facultativement substitué avec n'importe quelle combinaison accessible d'un ou plusieurs groupes R¹⁰ ou R⁷ ;
A représente H, un groupe cycloalkyle en C₃ à C₆, Het ou Ar ;
R⁷ représente un groupe -COR⁸, -COCR'₂R⁹, -C(S)R⁸, -S(O)ₘOR', -S(O)ₘNR'R", -PO(OR'), -PO(OR')₂, -NO₂ ou tétrazolyle ;
chaque groupe R⁸ représente indépendamment un groupe -OR', -NR'R", -NR'SO₂R', -NR'OR' ou -OCR'₂CO(O)R' ;
R⁹ représente un groupe -OR', -CN, -S(O)ᵣR', -S(O)ₘNR'₂, -C(O)R', C(O)NR'₂ ou -CO₂R' ;
R¹⁰ représente H, un groupe halogéno, -OR¹¹, -CN, -NR'R¹¹, -NO₂, -CF₃, CF₃S(O)ᵣ-, -CO₂R' , -CONR'₂, A-(alkyle en C₀ à C₆)-, A- (oxoalkyle en C₁ à C₆)-, A-(alcényle en C₂ à C₆)-, A-(alcynyle en C₂ à C₆)-, A-(alkyloxy en C₀ à C₆)-, A-(alkylamino en C₀ à C₆) - ou A-(alkyle en C₀ à C₆)-S(O)ᵣ- ;
R¹¹ représente un groupe R', -C(O)R', -C(O)NR'₂, -C(O)OR', -S'(O)ₘR' ou -S(O)ₘNR'₂ ;
R² représente un groupe 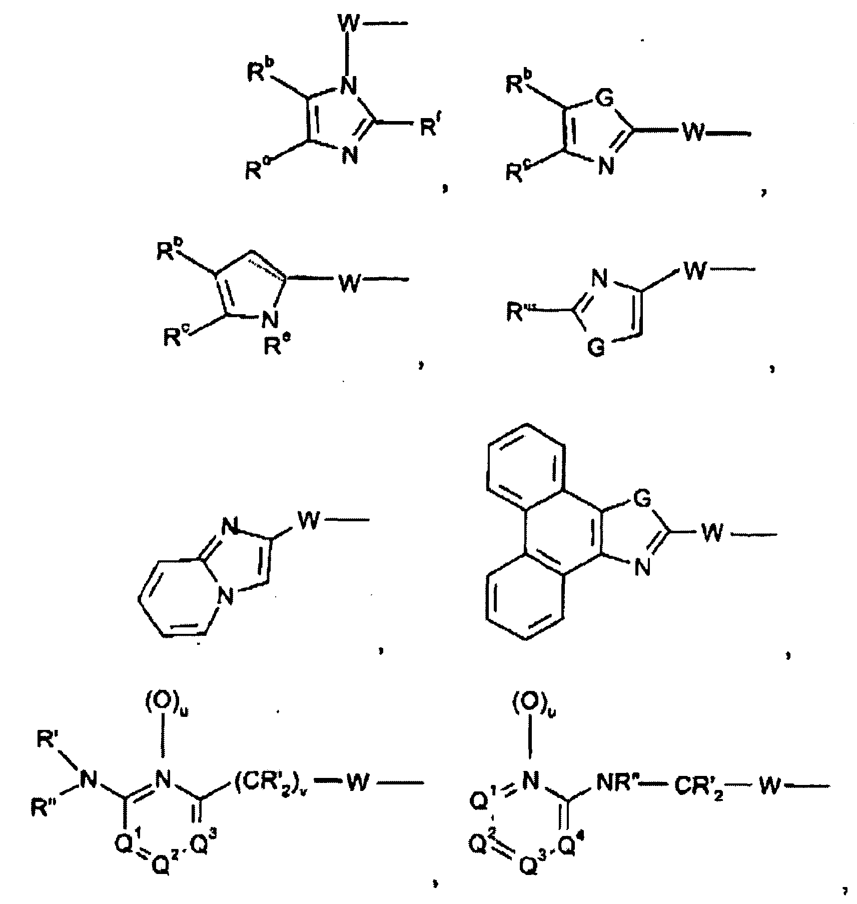 ou 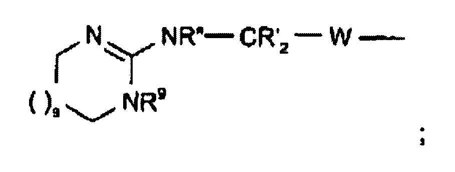
W représente -(CHR^{g})ₐ-U-(CHR^{g})_{b}- ;
U est absent ou représente un groupe CO, CR^{g}₂, C(=CR^{g}₂), S(O)ₖ, O, NR^{g}, CR^{g}OR^{g}, CR^{g}(OR^{k})CR^{g}₂, CR^{g}₂CR^{g}(OR^{k}), C(O)CR^{g}₂, CR^{g}2C(O), CONR¹, NRⁱCO, OC(O), C(O)O, C(S)0, OC(S), C(S)NR^{g}, NR^{g}C(S), S(O)₂NR^{g}, NR^{g}S(O)₂, N=N, NR^{g}NR^{g}, NR^{g}CR^{g}₂, CR^{g}₂NR^{g}, CR^{g}₂O, OCR^{g}₂, C≡C ou CR^{g}=CR⁹;
G représente un groupe NR^{e}, S ou 0 ;
R^{g} représente H, un groupe alkyle en C₁ à C₆, Het-(alkyle en C₀ à C₆), (cycloalkyle en C₃ à C₇)-(alkyle en C₀ à C₆) ou Ar-(alkyle en C₀ à C₆) ;
R^{k} représente un groupe R^{g}, -C(O)R^{g} ou -C(O)OR^{f} ;
Rⁱ représente H, un groupe alkyle en C₁ à C₆, Het-(alkyle en C₀ à C₆), (cycloalkyle en C₃ à C₇)-(alkyle en C₀ à C₆), Ar-(alkyle en C₀ à C₆) ou alkyle en C₁ à C₆ substitué avec un à trois groupes choisis entre des groupes halogéno, CN, NR^{g}₂, OR^{g}, SR^{g}, CO₂R^{g} et CON(R^{g})₂ ;
R^{f} représente H, un groupe alkyle en C₁ à C₆ ou Ar-(alkyle en C₀ à C₆) ;
R^{e} représente H, un groupe alkyle en C₁ à C₆, Ar-(alkyle en C₀ à C₆), Het-(alkyle en C₀ à C₆), (cycloalkyle en C₃ à C₇)-(alkyle en C₀ à C₆) ou (CH₂)ₖCO₂R^{g} ;
R^{b} et R^{c} sont choisis indépendamment entre H, des groupes alkyle en C₁ à C₆, Ar-(alkyle en C₀ à C₆), Het-(alkyle en C₀ à C₆) et (cycloalkyle en C₃ à C₆)-(alkyle en C₀ à C₆), halogéno, CF₃, OR^{f}, S(O₎ₖR^{f}, COR^{f}, NO₂, N(R^{f})₂, CO(NR^{f})₂, CH₂N(R^{f})₂, ou bien R^{b} et R^{c} sont joints l'un à l'autre pour former un noyau carbocyclique ou hétérocyclique aromatique ou non aromatique, penta- ou hexagonal, facultativement substitué avec jusqu'à trois substituants choisis entre des substituants halogéno, CF₃, alkyle en C₁ à C₄, OR^{f}, S(O)ₖR^{f}, COR^{f}, CO₂R^{f}, OH, NO₂, N(R^{f})₂, CO(NR^{f})₂ et CH₂N(R^{f})₂ ; et méthylènedioxy ;
Q¹, Q² Q³ et Q⁴ représentent indépendamment N ou un groupe C-R^{y}, sous réserve que pas plus d'un de Q¹, Q², Q³ et Q⁴ ne représente N ;
R' représente H, un groupe alkyle en C₁ à C₆, Ar-(alkyle en C₀ à C₆) ou (cycloalkyle en C₃ à C₆)-(alkyle en C₀ à C₆) ;
R" représente un groupe R', -C(O)R' ou -C(O)OR' ;
R''' représente H, un groupe alkyle en C₁ à C₆, Ar-(alkyle en C₀ à C₆), Het-(alkyle en C₀ à C₆) ou (cycloalkyle en C₃ à C₆)-(alkyle en C₀ à C₆), halogéno, CF₃, OR^{f}, S(O)ₖR^{f}, COR^{f}, NO₂, N(R^{f})₂, CO(NR^{f})₂, CH₂N(R^{f})₂ ;
R^{y} représente H, un groupe halogéno, -OR^{g}, -SR^{g}, -CN, -NR^{g}R^{k}, -NO₂, -CF₃, CF₃S(O)ᵣ-, -CO₂R^{g}, -COR^{g} ou -CONR^{g}₂, ou alkyle en C₁ à C₆ facultativement substitué avec un substituant halogéno, -OR^{g}, -SR^{g}, -CN, -NR^{g}R", -NO₂, -CF₃, R'S(O)ᵣ-, -CO₂R^{g}, -COR^{g} ou -CONR^{g}₂ ;
a est égal à 0, 1 ou 2 ;
b est égal à 0, 1 ou 2 ;
k est égal à 0, 1 ou 2 ;
m est égal à 1 ou 2 ;
r est égal à 0, 1 ou 2 ;
s est égal à 0, 1 ou 2 ;
u est égal à 0 ou 1 ; et
v est égal à 0 ou 1 ;
ou d'un de ses sels pharmaceutiquement acceptables,
dans la production d'un médicament destiné au traitement des adhérences postchirurgicales.

2. Utilisation suivant la revendication 1, dans laquelle le composé est l'acide (S)-3-oxo-8-[3-(pyridine-2-ylamino)-1-propyloxy]-2-(2,2,2-trifluorométhyl)-2,3,4,5-tétrahydro-1H-2-benzazépine-4-acétique ou un de ses sels pharmaceutiquement acceptables.

3. Utilisation suivant la revendication 1, dans laquelle le composé est l'acide (S)-8-[2-[6-(méthylamino)-pyridine-2-yl]-1-éthoxy]-3-oxo-2-(2,2,2-trifluoréthyl)-2,3,4,5-tétrahydro-1H-2-benzazépine-4-acétique ou un de ses sels pharmaceutiquement acceptables.

4. Utilisation de l'acide (S)-10,11-dihydro-3-[2-(5,6,7,8-tétrahydro-1,8-naphtyridine-2-yl)-1-éthoxy]-5H-dibenzo[a,d]cycloheptène-10-acétique, ou d'un de ses sels pharmaceutiquement acceptables, dans la production d'un médicament destiné au traitement des adhérences postchirurgicales.
